# EUROPÄISCHE PATENTSCHRIFT

(11) **EP 1 268 411 B1**
(45) Veröffentlichungstag und Bekanntmachung des Hinweises auf die Patenterteilung: **19.12.2007**
(21) Anmeldenummer: 01900112.2
(22) Anmeldetag: 03.01.2001
(51) Int. Cl.: C07C 277/08, C07C 279/36

(54) **VERFAHREN ZUR HERSTELLUNG VON N-METHYL-N'-NITROGUANIDIN**
METHOD FOR PRODUCING N-METHYL-N'-NITROGUANIDINE
PROCEDE DE PREPARATION DE N-METHYL-N'-NITROGUANIDINE

(30) Priorität: 12.01.2000 DE 10000891
(43) Veröffentlichungstag der Anmeldung: 02.01.2003
(73) Patentinhaber: Bayer CropScience AG, 40789 Monheim (DE)
(72) Erfinder: VAN LAAK, Kai, 51061 Köln (DE); SIRGES, Wolfram, 40597 Düsseldorf (DE)
(86) Internationale Anmeldenummer: PCT/EP2001/000015
(87) Internationale Veröffentlichungsnummer: WO 2001/051458

(56) Entgegenhaltungen:
- EP-A- 0 798 293
- WO-A-00/31025

## Beschreibung

Die Erfindung betrifft ein neues Verfahren zur Herstellung von N-Methyl-N'-nitroguanidin.

Es ist bekannt, dass man N-Methyl-N'-nitroguanidin erhält, wenn man zunächst die Verbindung der Formel (A) in üblicher Weise nitriert und anschließend in einer zweiten Reaktionsstufe die Methylmercaptogruppe gegen Methylamin gemäß dem folgenden Reaktionsschema austauscht: 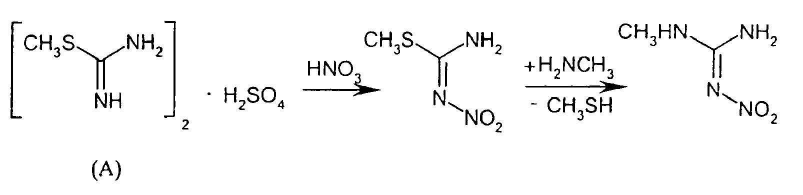 (vgl. hierzu JACS 1954, 76, 1877).

Dieses Verfahren hat jedoch den Nachteil, dass es sich um eine zweistufige Umsetzung handelt. Wenngleich die Ausbeuten in beiden Stufen verhältnismäßig gut sind, wirft die Abspaltung von Methylmercaptan, vor allem bei einer Durchführung im großtechnischen Maßstab, verfahrenstechnische Probleme auf.

Ferner ist bekannt, dass das N-Methyl-N'-nitroguanidin erhalten werden kann, wenn man eine alkalische Lösung (Kaliumhydroxid) von Nitroguanidin mit Methylamin-Hydrochlorid bei 60°C gemäß dem folgenden Reaktionsschema umsetzt: 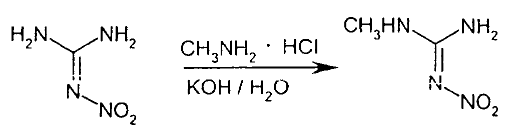 (vgl. hierzu JACS 1947, 69, 3028).

Dieses Verfahren hat jedoch den Nachteil, dass zum Erhalt eines sauberen Produkts mindestens ein bis zwei Umkristallisationen zur Entfernung anorganischer Verunreinigungen notwendig sind, was zu Ausbeuteverlusten führt.

In EP 0 798 293 wird ein Verfahren beschrieben, wodurch man N-Methyl-N'-nitroguanidin erhält, wenn man Nitroguanidin mit wässriger Methylaminlösung bei Temperaturen zwischen 0°C und 40°C umsetzt.

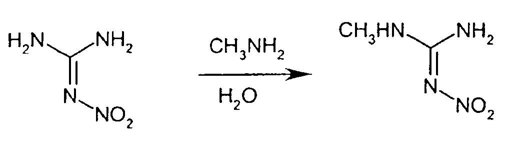

In diesem Verfahren sind jedoch die Reaktionszeiten sehr lang, so dass nur kleine Raum-Zeit-Ausbeuten erzielt werden. Diese geringe Raum-Zeit-Ausbeute wirft bei einer Durchführung im technischen Maßstab Probleme auf.

Es wurde nun gefunden, dass man N-Methyl-N'-nitroguanidin der Formel (I) 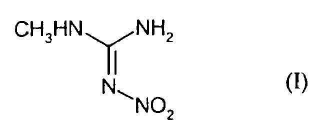 erhält,
wenn man Nitroguanidin in der Formel (II) 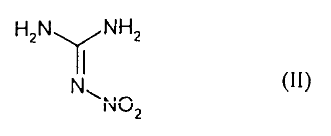 mit wässriger, durch Zugabe anorganischer oder organischer Säuren gepufferte Methylaminlösung umsetzt.

Überraschenderweise kann nach dem erfindungsgemäßen Verfahren das N-Methyl-N-nitroguanidin der Formel (I) auf einfache Art in sehr guter Raum-Zeit-Ausbeute und in hoher Reinheit erhalten werden. Nach dem Stand der Technik war nicht zu erwarten, dass die Reaktion durch Säurezugabe zu beschleunigen ist, da gemäß JACS 1947, 69, 1947 ein Überschuss an KOH verwendet wird, um Methylnitroguanidin zu erhalten.

Die erfindungsgemäße Umsetzung hat somit den Vorteil einer erhöhten Reaktionsgeschwindigkeit. Dies führt zu dem technischen Vorteil der hohen Raum-Zeit-Ausbeute.

Der Reaktionsablauf des erfindungsgemäßen Verfahrens kann durch das folgende Reaktionsschema skizziert werden:

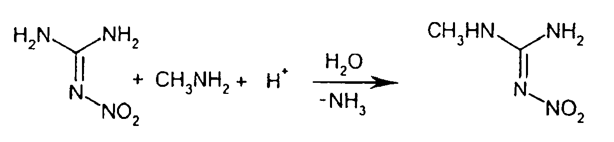

Die Ausgangsstoffe Nitroguanidin der Formel (II) und Methylamin sind allgemein bekannte Verbindungen der organischen Chemie.

Zur Pufferung der Reaktionslösung können alle üblichen anorganischen oder organischen Säuren verwendet werden. Beispielhaft genannt seien Salzsäure, Schwefelsäure, Salpetersäure, Phosphorsäure, Essigsäure, Ameisensäure und Trifluoressigsäure.

Das erfindungsgemäße Verfahren wird in Gegenwart von Wasser ais Verdünnungsmittel durchgeführt. Es ist aber auch möglich, im organisch/wässrigen System zu arbeiten, wobei alle üblichen, mit Wasser mischbaren organischen Lösungsmittel eingesetzt werden können. Beispielhaft genannt seien Ketone, wie Aceton, Methylethyl-keton oder Methyl-isobutyl-keton; Nitrile, wie Acetonitril oder Propionitril sowie Alkohole, wie Methanol oder Ethanol.

In der WO 00/31025 mit Veröffentlichungsdatum vom 2.6.2000 wird ein derartiges Verfahren mit Reaktionstemperaturen > 60ºC beschrieben. Die Reaktionstemperaturen können bei der Durchführung des erfindungsgemäßen Verfahrens in einem größeren Bereich variiert werden. Im allgemeinen arbeitet man bei Temperaturen zwischen 0°C und 40°C, bevorzugt zwischen 10°C und 30°C, besonders bevorzugt zwischen 20°C und 30°C.

Bei der Durchführung des erfindungsgemäßen Verfahrens setzt man pro Mol Nitroguanidin der Formel (II) im allgemeinen 1 bis 3 Mol, vorzugsweise 1 bis 2 Mol Methylamin ein.

Im erfindungsgemäßen Verfahren wird der pH-Wert durch den Zusatz von Säure im allgemeinen auf pH = 11,0 bis 13,0, vorzugsweise auf pH = 11,5 bis 13,0 eingestellt.

Die Aufarbeitung kann auf übliche Art und Weise durchgeführt werden.

Das nach dem erfindungsgemäßen Verfahren herzustellende N-Methyl-N'-nitroguanidin der Formel (I) kann als Zwischenprodukt zur Herstellung von biologisch aktiven Verbindungen, beispielsweise von Insektiziden verwendet werden (vgl. z.B. EP-A 0 376 279 und EP-A 0 428 941).

### Herstellungsbeispiel

### Beispiel 1

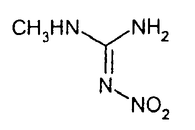

65 g (0,5 mol; 80 %ig wasserfeucht) Nitroguanidin werden in 75 ml Wasser vorgelegt und auf 5°C gekühlt. 59 g (0,75 mol) 40 %ige wässrige Methylaminlösung werden zudosiert. Anschließend werden 24,8 g (0,1 mol) 20 %ig Schwefelsäure zudosiert. Die Mischung wird auf 25°C erwärmt und 8 Stunden lang bei dieser Temperatur gerührt. Es wird auf 5°C abgekühlt, filtriert und mit 50 ml Wasser gewaschen.

Man erhält 51,3 g (87 % der Theorie) N-Methyl-N'-nitroguanidin vom Schmelzpunkt 160°C mit einem Gehalt von 99 % (nach HPLC).

## Patentansprüche

1. Verfahren zur Herstellung von N-Methyl-N'-nitroguanidin der Formel (I) 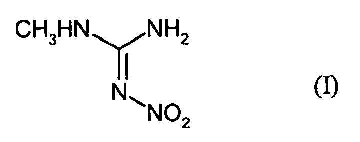 **dadurch gekennzeichnet, dass** man Nitroguanidin der Formel (II) 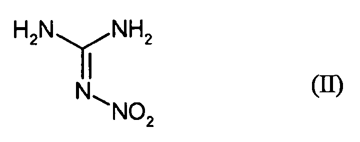 mit wässriger, durch Zugabe anorganischer oder organischer Säuren gepufferter Methylaminlösung, bei Temperaturen < 60°C umsetzt.

2. Verfahren zur Herstellung von N-Methyl-N'-nitroguanidin der Formel (I) 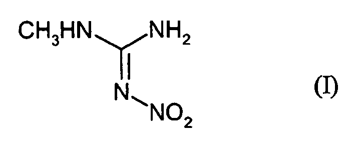 gemäß Anspruch 1, **dadurch gekennzeichnet, dass** die Reaktionstemperatur im Bereich zwischen 0°C und 40°C liegt.

3. Verfahren zur Herstellung von N-Methyl-N'-nitroguanidin der Formel (I) 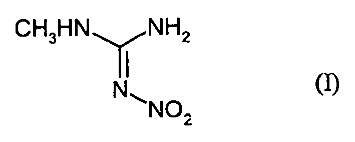 gemäß Anspruch 1, **dadurch gekennzeichnet, dass** die Reaktionstemperatur im Bereich zwischen 10°C und 30°C liegt.

4. Verfahren zur Herstellung von N-Methyl-N'-nitroguanidin der Formel (I) 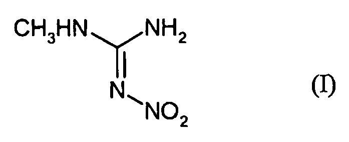 gemäß Anspruch 1, **dadurch gekennzeichnet, dass** die Reaktionstemperatur im Bereich zwischen 20°C und 30°C liegt.

5. Verfahren zur Herstellung von N-Methyl-N'-nitroguanidin der Formel (I) 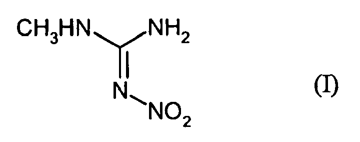 gemäß Anspruch 1, **dadurch gekennzeichnet, dass** der pH Wert zwischen 11 und 13 liegt.

6. Verfahren zur Herstellung von N-Methyl-N'-nitroguanidin der Formel (I) 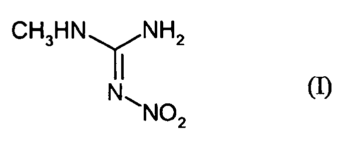 gemäß Anspruch 1, **dadurch gekennzeichnet, dass** der pH Wert zwischen 11,5 und 13 liegt.

7. Verfahren zur Herstellung von N-Methyl-N'-nitroguanidin der Formel (I) 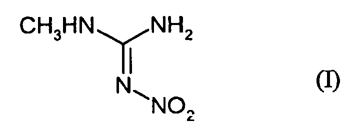 gemäß Anspruch 6, **dadurch gekennzeichnet, dass** die Reaktionstemperatur Reaktionstemperatur im Bereich zwischen 0°C und 40°C liegt.

8. Verfahren zur Herstellung von N-Methyl-N'-nitroguanidin der Formel (I) 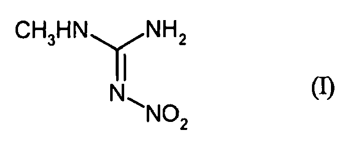 gemäß Anspruch 6, **dadurch gekennzeichnet, dass** die Reaktionstemperatur Reaktionstemperatur im Bereich zwischen 20°C und 40°C liegt.

## Claims

1. Process for the preparation of N-methyl-N'-nitroguanidine of the formula (I) 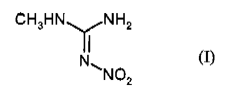 **characterized in that** nitroguanidine of the formula (II) 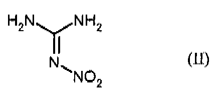 is reacted with aqueous methylamine solution buffered by the addition of inorganic or organic acids at temperatures of <60°C.

2. Process for the preparation of N-methyl-N'-nitroguanidine of the formula (I) 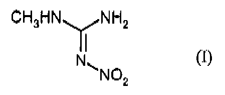 according to Claim 1, **characterized in that** the reaction temperature is in the range between 0°C and 40°C.

3. Process for the preparation of N-methyl-N'-nitroguanidine of the formula (I) 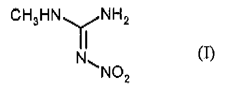 according to Claim 1, **characterized in that** the reaction temperature is in the range between 10°C and 30°C.

4. Process for the preparation of N-methyl-N'-nitroguanidine of the formula (1) 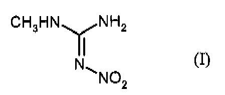 according to Claim 1, **characterized in that** the reaction temperature is in the range between 20°C and 30°C.

5. Process for the preparation of N-methyl-N'-nitroguanidine of the formula (I) 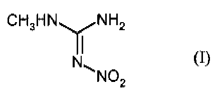 according to Claim 1, **characterized in that** the pH is between 11 and 13.

6. Process for the preparation of N-mothyl-N'-nitroguanidine of the formula (I) 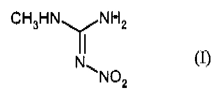 according to Claim 1, **characterized in that** the pH is between 11.5 and 13.

7. Process for the preparation of N-methyl-N'-nitroguanidine of the formula
(I) 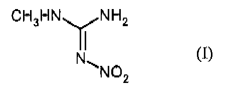 according to Claim 6, **characterized in that** the reaction temperature is in the range between 0°C and 40°C.

8. Process for the preparation of N-methyl-N'-nitroguanidine of the formula (I) 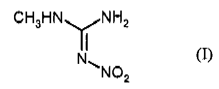 according to Claim 6, **characterized in that** the reaction temperature is in the range between 20°C and 40°C.

## Revendications

1. Procédé de production de N-méthyl-N'-nitroguanidine de formule (I) 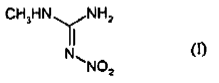 **caractérisé en ce qu'**on fait réagir de la nitroguanidine de formule (II) 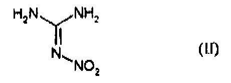 avec une solution aqueuse de méthylamine tamponnée par addition d'acides inorganiques ou organiques, à des températures inférieures à 60°C.

2. Procédé de production de N-méthyl-N'-nitroguanidine de formule (I) 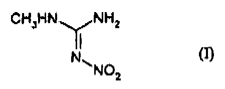 selon la revendication 1, **caractérisé en ce que** la température de réaction se situe dans la plage allant de 0°C à 40°C.

3. Procédé de production de N-méthyl-N'-nitroguanidine de formule (I) 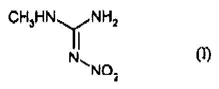 selon la revendication 1, **caractérisé en ce que** la température de réaction se situe dans la plage allant de 10°C à 30°C.

4. Procédé de production de N-méthyl-N'-nitroguanidine de formule (I) 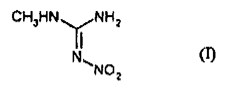 selon la revendication 1, **caractérisé en ce que** la température de réaction se situe dans la plage allant de 20°C à 30°C.

5. Procédé de production de N-méthyl-N'-nitroguanidine de formule (I) 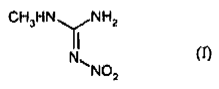 selon la revendication 1, **caractérisé en ce que** la valeur du pH se situe entre 11 et 13.

6. Procédé de production de N-méthyl-N'-nitroguanidine de formule (I) 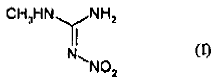 selon la revendication 1, **caractérisé en ce que** la valeur du pH se situe entre 11,5 et 13.

7. Procédé de production de N-méthyl-N'-nitroguanidine de formule (I) 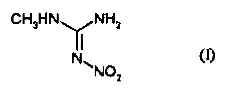 selon la revendication 6, **caractérise en ce que** la température de réaction se situe dans la plage allant de 0°C à 40°C.

8. Procédé de production de N-méthyl-N'-nitroguanidine de formule (I) 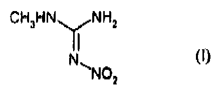 selon la revendication 6, **caractérisé en ce que** la température de réaction se situe dans la plage allant de 20°C à 40°C.
